# EUROPÄISCHE PATENTSCHRIFT

(11) **EP 1 600 190 B1**
(45) Veröffentlichungstag und Bekanntmachung des Hinweises auf die Patenterteilung: **29.08.2007**
(21) Anmeldenummer: 05010397.7
(22) Anmeldetag: 12.05.2005
(51) Int. Cl.: A61M 5/31, A61M 5/32

(54) **Spritzenverschluss und Verfahren zum Herstellen eines Spritzenverschlusses**
Syringe cap and method of producing a syringe cap
Capuchon de seringue et méthode de fabrication d'un capuchon de seringue

(30) Priorität: 29.05.2004 EP 04012833
(43) Veröffentlichungstag der Anmeldung: 30.11.2005
(73) Patentinhaber: Gerresheimer Bünde GmbH, 32257 Bünde (DE)
(72) Erfinder: Geiger, Andreas, 56841 Traben-Trarbach (DE); Wittland, Frank, 32130 Enger (DE)
(74) Vertreter: Schirmer, Siegfried

(56) Entgegenhaltungen:
- EP-A- 0 876 824
- EP-A- 1 192 965
- DE-A1- 19 956 243
- DE-C1- 4 140 101

## Beschreibung

Die Erfindung betrifft einen Spritzenverschluss gemäß dem Oberbegriff des Anspruchs 1 zum abdichtenden Verschließen einer distalen Öffnung eines Spritzenkörpers sowie ein Verfahren zum Herstellen eines solchen Spritzenverschlusses.

In US 6,190,364 wird ein derartiger Spritzenverschluss für einen Spritzenkörper beschrieben. Der Spritzenkörper weist eine distale Spitze und eine sich durch diese erstreckende distale Öffnung auf, so dass eine Spritzenflüssigkeit, die sich in dem Spritzenkörper befindet, durch die distale Öffnung den Spritzenkörper verlassen kann. Die Spritze umfasst einen Befestigungsring (engl. "luer collar"), der einteilig mit der Spritze ausgebildet oder auf der distalen Spitze des Spritzenkörpers aufgesetzt und fest damit verbunden ist. Der Befestigungsring weist Gewindeelemente auf, die mit Gewindeelementen einer zweiteiligen Verschlusskappe zusammenwirken, um die Verschlusskappe am Befestigungsring zu halten. Die Verschlusskappe umschließt im Gebrauch die distale Spitze und verschließt und dichtet die distale Öffnung des Spritzenkörpers ab.

Zum Injizieren der Spritzenflüssigkeit wird die Verschlusskappe aus dem Befestigungsring geschraubt und eine Spritzennadel wird an dem Befestigungsring und damit an dem Spritzenkörper so befestigt, dass eine sich durch die Spritzennadel erstreckende Nadelöffnung in Strömungsverbindung mit der distalen Öffnung des Spritzenkörpers steht. Zum Befestigen der Spritzennadel an dem Befestigungsring ist die Spritzennadel mit einem Gewindeelement verbunden, das mit dem Gewindeelement des Befestigungsrings zusammenwirkt.

Um mögliche Manipulationen an der Spritzenfüllung erkennbar zu machen, weist der Spritzenverschluss gemäß US 6,190,364 einen Versiegelungsstreifen auf, der sowohl mit dem Befestigungsring als auch mit der Verschlusskappe unlösbar verbunden ist, so dass der Versiegelungsstreifen reißt, wenn die Verschlusskappe von dem Befestigungsring gelöst wird.

Nachteilig ist bei diesem Spritzenverschluss, dass die Herstellung dreier separater Teile (zweiteilige Verschlusskappe, Versiegelungselement) und das Zusammenfügen dieser Teile zu einem Spritzenverschluss aufwendig ist.

Es ist daher die Aufgabe der Erfindung, einen Spritzenverschluss zum abdichtenden Verschließen einer distalen Öffnung eines Spritzenkörpers bereitzustellen, bei dem Manipulationen an der Spritzenfüllung erkannt werden können und der konstruktiv bzw. in der Herstellung vereinfacht ist.

Diese Aufgabe wird gelöst durch einen Spritzenverschluss nach Anspruch 1 zum abdichtenden Verschließen einer distalen Öffnung eines Spritzenkörpers, wobei der Spritzenverschluss einen an dem Spritzenkörper um die distale Öffnung herum anordbaren und befestigbaren Befestigungsring und eine die distale Öffnung abdichtend verschließende und mit dem Befestigungsring lösbar verbundene Verschlusskappe aufweist, wobei der Befestigungsring ein Rastmittel umfasst, das mit einem Rastmittel der Verschlusskappe verrastet ist, und wobei das Rastmittel des Befestigungsrings und das Rastmittel der Verschlusskappe so ausgebildet sind, dass sie nicht beschädigungsfrei entrastbar sind, d. h. nach Entrasten ist keine beschädigungsfreie Wiederverrastung möglich.

Es kann vorgesehen sein, dass die Rastmittel durch Rastabschnitte und diese formschlüssig umschließende Rastaussparungen gebildet sind, wobei die Rastabschnitte entweder mit dem Befestigungsring oder mit der Verschlusskappe und die Rastaussparungen mit dem anderen Teil, Befestigungsring oder Verschlusskappe, verbunden sind, so dass die Verschlusskappe mit dem Befestigungsring verrastet ist und nicht beschädigungsfrei entrastbar ist.

Die Rastaussparungen können in Längsrichtung hinterschnitten ausgebildet sein. Des Weiteren können die Rastaussparungen in radialer Richtung hinterschnitten ausgebildet sein.

Bevorzugt weist der Befestigungsring einen proximalen, ringförmigen Abschnitt und eine sich distal von dem ringförmigen Abschnitt erstreckende, hohlzylinderförmige Befestigungswand auf.

Zweckmäßiger Weise kann vorgesehen sein, dass die Rastabschnitte mit einer äußeren Ringwulst der Verschlusskappe verbunden sind und sich ausgehend von der Ringwulst parallel zur Längsachse, in proximaler Richtung und mit Abstand zu einer Außenfläche der Verschlusskappe erstrecken, wobei die Rastaussparungen zu einer distalen, stimseitigen Endfläche der Befestigungswand hin geöffnet sind.

Es kann vorgesehen sein, dass in der Befestigungswand Rastaussparungen ausgebildet sind, die auf einer Außenfläche der Verschlusskappe ausgebildete Rastabschnitte formschlüssig umschließen. Der Befestigungsring und die Verschlusskappe können miteinander zusammenwirkende Gewindeelemente aufweisen, die eine Gewindesteigung festlegen.

Zweckmäßigerweise weist jeder Rastabschnitt eine unter einem Steigungswinkel verlaufende Anschlagfläche auf, die bei einem durch die Gewindeelemente geführten Herausdrehen der Verschlusskappe aus dem Befestigungsring gegen eine Seitenfläche der den jeweiligen Rastabschnitt umschließenden Rastaussparung anliegt, wobei der Steigungswinkel größer ist als die Gewindesteigung, so dass die Rastabschnitte und/oder die Rastaussparungen beim Herausdrehen zwangsläufig deformiert werden.

Der Befestigungsring kann auf eine distale Spitze des Spritzenkörpers, durch die sich die distale Öffnung erstreckt, aufsetzbar und darauf fixierbar sein.

Bevorzugt weist die Verschlusskappe zum Aufnehmen einer distalen Spitze des Spritzenkörpers die Form eines einseitig geschlossenen Hohlzylinders auf.

Zweckmäßigerweise ist in der Verschlusskappe ein zylinderförmiger Verschlusspfropfen angeordnet, auf der ein mittiger, vorstehender Abschnitt zum Eingreifen in die distale Öffnung des Spritzenkörpers angeordnet ist. Bei einer bevorzugten Ausführung der Erfindung weist die Verschlußkappe einen der distalen Öffnung des Spritzenkörpers zugeordneten mittigen Abschnitt zum Eingreifen in die distale Öffnung auf.

Die Verschlußkappe kann äußere Längsrippen aufweisen und aus thermoplastischen Elastomeren gebildet sein.

Zweckmäßigerweise sind der Befestigungsring und die Verschlusskappe in einem Zweikomponenten-Spritzverfahren aus unterschiedlichen, sich nicht einstückig miteinander verbindenden Materialien hergestellt.

Die Aufgabe wird ferner gelöst durch ein Verfahren gemäß Anspruch 17 zum Herstellen eines erfindungsgemäßen Spritzenverschlusses, bei dem die Verschlusskappe und der Befestigungsring zusammen mittels eines Zweikomponenten-Spritzverfahrens so ineinander verrastet hergestellt werden, dass sie nicht beschädigungsfrei entrastbar sind und nach Entrasten nicht beschädigungsfrei wieder verrastbar sind.

Bevorzugt wird nach der Herstellung der Verschlusskappe und des Befestigungsrings ein Verschlusspfropfen in die Verschlusskappe eingesetzt.

Bei einer vorteilhaften Ausbildung erfolgt die Abdichtung des Spritzenkörpers durch die Verschlußkappe, die hierzu einen der distalen Öffnung des Spritzenkörpers zugeordneten Abschnitt zum Eingreifen in die distale Öffnung aufweist. Hierdurch ist eine zuverlässige Abdichtung ohne den erwähnten Verschlusspfropfen garantiert. Die Verschlußkappe ist aus thermoplastischen Elastomeren gebildet.

Die Erfindung wird im Folgenden anhand von Zeichnungen näher erläutert. Es zeigen:
- Fig. 1: eine Seitenansicht einer ersten Ausführungsform eines erfindungsgemäßen Spritzenverschlusses, aufgesetzt auf einen Spritzenkörper,
- Fig. 2: eine Längsschnittsansicht des Spritzenverschlusses aus Fig.1 entlang der Linie II-II in Fig. 1,
- Fig. 3: eine Seitenansicht einer zweiten Ausführungsform des erfindungsgemäßen Spritzenverschlusses, aufgesetzt auf einen Spritzenkörper,
- Fig. 4: eine Längsschnittsansicht des Spritzenverschlusses aus Fig. 3 entlang der Linie IV-IV in Fig. 3,
- Fig. 5: eine Längsschnittsansicht des Spritzenverschlusses aus den Figuren 3 und 4 entlang der Linie V-V in Fig. 3,
- Fig. 6: eine Seitenansicht einer weiteren Ausführungsform des erfindungsgemäßen Spritzenverschluss, aufgesetzt auf einen Spritzenkörper,
- Fig. 7: eine Querschnittsansicht des Spritzenverschlusses aus Fig. 6 entlang der Linie VII-VII in Fig. 6,
- Fig. 8: eine Längsschnittsansicht des Spritzenverschlusses aus den Figuren 6 und 7 entlang der Linie VIII-VIII in Fig. 6,
- Fig. 9: eine Seitenansicht einer weiteren Ausführungsform des erfindungsgemäßen Spritzenverschlusses, aufgesetzt auf einen Spritzenkörper,
- Fig. 10: eine Längsschnittsansicht des Spritzenverschlusses aus Fig. 9 entlang der Linie X-X in Fig. 9,
- Fig. 11: eine Querschnittsansicht des Spritzenverschlusses aus den Figuren 9 und 10 entlang der Linie XI-XI in Fig. 9 und
- Fig. 12: eine Längsschnittansicht einer Variante der Abdichtung des Spritzenkörpers.

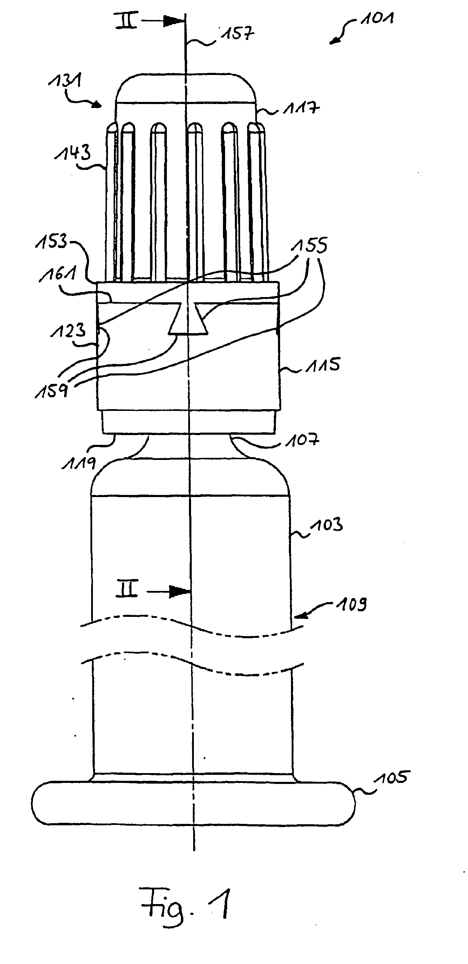

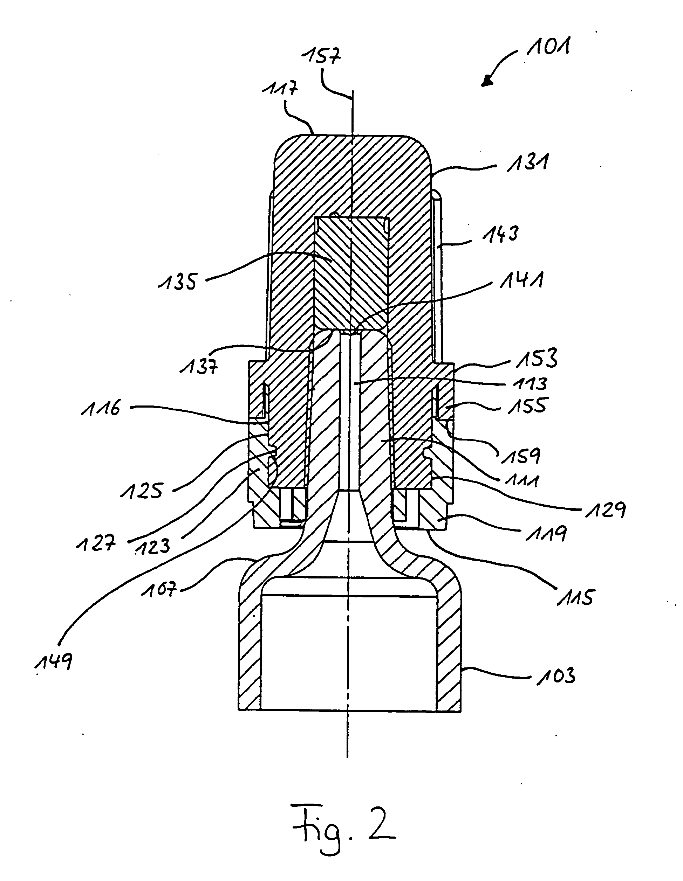

Fig. 1 und 2 zeigen in einer ersten Ausführungsform der Erfindung einen erfindungsgemäßen Spritzenverschluss 101, der auf einen Spritzenkörper 103 aufgesetzt ist. Der Spritzenkörper 103 weist einen proximalen 105 und einen distalen Endabschnitt 107 auf, die durch einen Hohlzylinder 109, der eine Spritzenflüssigkeit enthält (nicht dargestellt), verbunden sind. Der distale Endabschnitt 107 ist mit einer distalen Spitze 111 versehen, in der eine distale Öffnung 113 ausgebildet ist.

Der Spritzenverschluss 101 umfasst zunächst einen üblicherweise als Luer-Lock-Ring oder Luer-Lock-Adapter bezeichneten Befestigungsring 115 und eine Verschlusskappe 117, die mit dem Befestigungsring 115 nicht zerstörungsfrei lösbar verbunden ist, wie noch im einzelnen erläutert wird. Der Befestigungsring 115 weist einen proximalen, ringförmigen Abschnitt 119 auf, der im auf die distale Spitze 111 des Spritzenkörpers 103 aufgesetzten Zustand durch Reibungskräfte festsitzend auf der Spitze gehalten ist. Dazu kann ein äußerer ringförmiger Abschnitt radial nach innen vorstehende Anlageabschnitte aufweisen, die eine optimale Fixierung des Befestigungsrings an der Spitze ermöglichen. Des Weiteren kann der Spritzenkörper im Bereich der distalen Spitze eine äußere Ringwulst (nicht dargestellt) aufweisen, die der Befestigungsring formschlüssig umgreifen kann, was zu einer weiter verbesserten Fixierung führt. Dies ist an sich bekannt, so dass die Befestigung des Befestigungsrings am Spritzenkörper hier nicht weiter beschrieben wird.

An den ringförmigen Abschnitt 119 des Befestigungsrings 115 schließt sich distal eine hohlzylinderförmige Befestigungswand 123 an, deren Innenfläche 125 Gewindeelemente 127 trägt.

Die Verschlusskappe 117 hat die Form eines einseitig geschlossenen Hohlzylinders, wobei im aufgesetzten Zustand die distale Spitze 111 des Spritzenkörpers 103 innerhalb des Hohlzylinders angeordnet ist. Die Verschlusskappe 117 weist einen proximalen 129 und einen distalen Abschnitt 131 auf. In die Verschlusskappe 117 ist ein Verschlusspfropfen 135 eingelassen, der an einer proximalen Endfläche 137 einen mittigen vorstehenden Abschnitt 141 aufweist, der im aufgesetzten Zustand in die distale Öffnung 113 der distalen Spitze 111 greift, um den Spritzenkörper 103 zuverlässig abzudichten. Der Verschlusspfropfen kann einstückig mit der Verschlusskappe ausgebildet sein.

Der proximale Abschnitt 129 der Verschlusskappe 117 weist zu den Gewindeelementen 127 des Befestigungsrings 115 komplementäre Gewindeelemente 149 auf, um die Verschlusskappe 117 in dem Befestigungsring 115 zu halten. Alternativ zu Gewindeelementen kann der proximale Abschnitt 129 der Verschlusskappe 117 nach außen vorstehende Eingriffsabschnitte aufweisen, die in Gewindeelemente des Befestigungsrings 115 greifen, um die Verschlusskappe 117 in dem Befestigungsring 115 zu halten, oder umgekehrt könnte der Befestigungsring von seiner Befestigungswand nach innen vorstehende Eingriffsabschnitte aufweisen, die mit Gewindeelementen der Verschlusskappe zusammenwirken.

Außen auf dem distalen Abschnitt 131 der Verschlusskappe 117 sind Längsrippen 143 ausgebildet, die die Griffigkeit beim Herausdrehen der Verschlusskappe 117 aus dem Befestigungsring 115 verbessern.

Die Verschlusskappe 117 weist eine umlaufende, flanschartig vorstehende Ringwulst 153 auf, von der vier Rastabschnitte 155 (alternativ können auch mehr oder weniger Rastabschnitte ausgebildet sein) in proximaler Richtung abgehen, parallel zu einer Längsachse 157 und mit einem radialen Abstand zu einer Außenfläche 116 der Verschlusskappe 117. Die Rastabschnitte 155 sind in radialer Ansicht (Fig. 1) dreieckig und weisen somit mit zunehmendem axialen Abstand von der Ringwulst 153 einen zunehmenden Querschnitt auf, wobei benachbarte Rastabschnitte 155 jeweils einen gleichen Winkelabstand zueinander haben.

In der Befestigungswand 123 des Befestigungsrings 115 sind Rastaussparungen 159 ausgebildet, die die Rastabschnitte 155 formschlüssig umschließen. Die Rastaussparungen sind zu einer distalen, stirnseitigen Endfläche 161 hin offen, und der Querschnitt der Rastaussparungen 159 vergrößert sich mit zunehmendem axialen Abstand von der Endfläche 161, d.h. die Rastaussparungen sind in Längsrichtung hinterschnitten. Aufgrund der beschriebenen Formgebung sind die Rastabschnitte 155 mit den Rastaussparungen 159 gewissermaßen verrastet. Die Rastaussparungen 159 sind zudem zur Außenfläche der Befestigungswand 123 hin geöffnet, so dass die Rastabschnitte 155 und insbesondere ihre Verbindung mit der Ringwulst 153 für einen Benutzer von außen sichtbar sind. Von außen sichtbare Außenflächen der Rastabschnitte 155 sind ferner bündig mit der Außenfläche der Befestigungswand 123.

Die oben beschriebene Form der Rastabschnitte 155 und Rastaussparungen 159 führt dazu, dass die Verschlusskappe 117 nicht aus dem Befestigungsring 115 geschraubt werden kann, ohne dass die Rastabschnitte 155 und/oder Rastaussparungen 159 beschädigt werden. Entweder reißen die Rastabschnitte 155 beim Herausdrehen von der Verschlusskappe 117 ab, oder die Rastabschnitte 155 und/oder die Rastaussparungen 159 werden beim Herausdrehen irreversibel deformiert. Eine derartige Beschädigung zeigt einem Benutzer an, dass der Spritzenkörper 103 schon einmal geöffnet worden ist und macht etwaige Manipulationen an der Spritzenfüllung erkennbar.

Die erfindungsgemäße Verschlusskappe 101 ist nicht herstellbar, indem der Befestigungsring 115 und die Verschlusskappe 117 zunächst getrennt voneinander, bspw. in einem bekannten Spritzverfahren, hergestellt und dann zusammengesetzt werden, da die Rastabschnitte 115 nicht beschädigungsfrei in die Rastaussparungen 159 einsetzbar sind. Daher werden die Verschlusskappe 117 und der Befestigungsring 115 gleichzeitig in einem an sich bekannten Zweikomponenten-Spritzverfahren unter Bildung des kompletten Spritzenverschlusses 101 hergestellt. Dabei müssen für die Verschlusskappe 117 und den Befestigungsring 115 Materialien verwendet werden, die sich während des Zweikomponenten-Spritzverfahrens nicht einstückig miteinander verbinden, um ein Herausdrehen der Verschlusskappe 117 aus dem Befestigungsring 115 im späteren Gebrauch zu ermöglichen. Die Verschlusskappe 117 kann daher bspw. aus einem thermoplastischen Elastomer und der Befestigungsring 115 aus Polykarbonat hergestellt sein. Nachdem der Befestigungsring 115 und die Verschlusskappe 117 ineinander verrastet in einem Zweikomponenten-Spritzverfahren gleichzeitig hergestellt worden sind, wird der Verschlusspfropfen 135 in die Verschlusskappe 117 eingesetzt.

Zur Ausführung einer Injektion wird die Verschlusskappe 117 aus dem Befestigungsring 115 geschraubt und eine Nadel, die mit einem Gewindeelement versehen ist, in den Befestigungsring 115 geschraubt, so dass der Spritzenkörper 103 durch die distale Öffnung 113 hindurch in Verbindung mit der Nadel steht. Anschließend kann die zu injizierende Flüssigkeit mit einem Spritzenkolben injiziert werden.

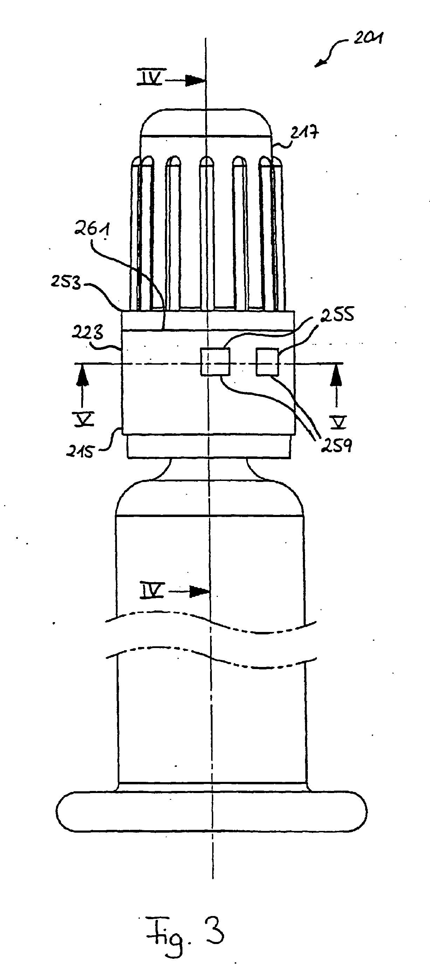

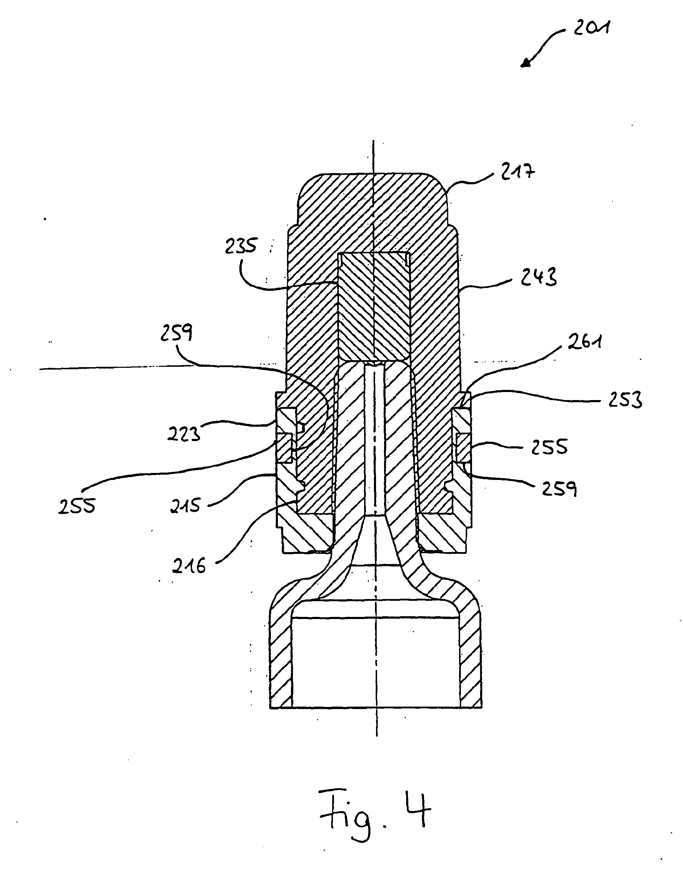

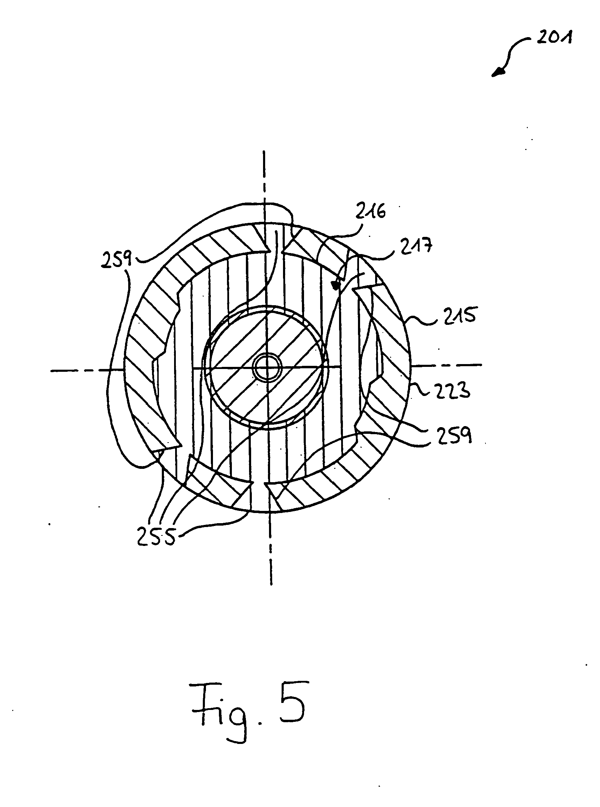

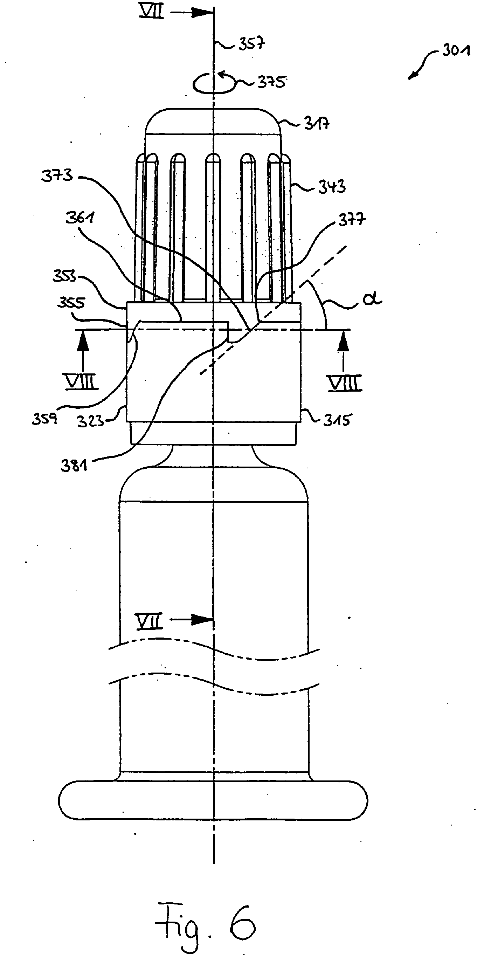

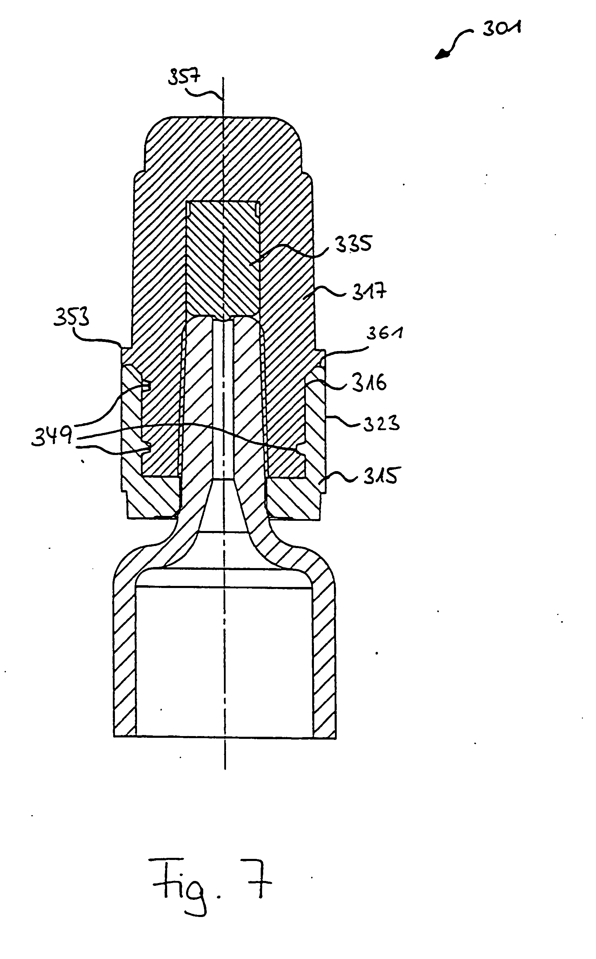

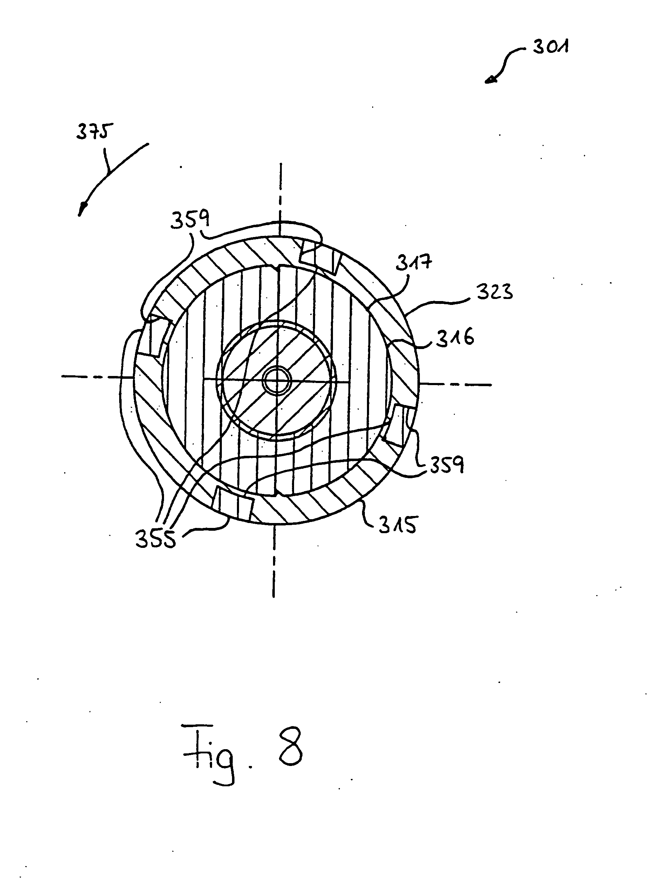

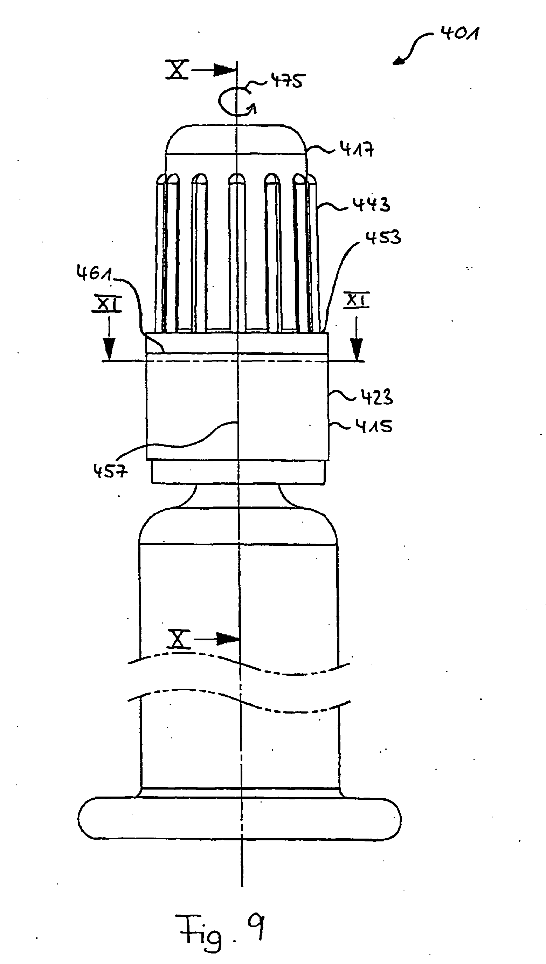

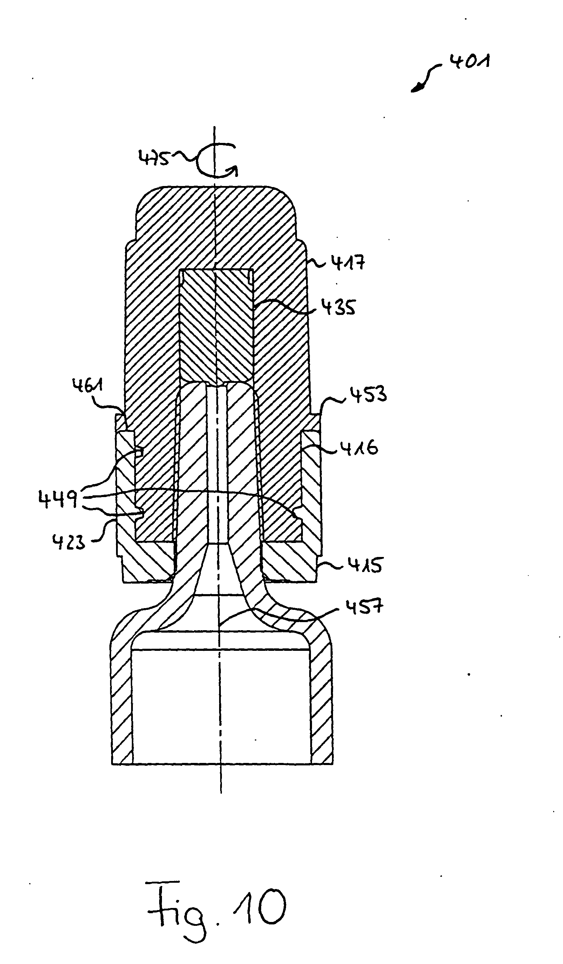

Fig. 3 bis 5 zeigen eine andere Ausführungsform eines Spritzenverschlusses 201 mit einem Befestigungsring 215, einer Verschlusskappe 217 und einem Verschlusspfropfen 235. Dieser Spritzenverschluss 201 unterscheidet sich von dem in Fig. 1 und 2 dargestellten Spritzenverschluss 101 durch anders ausgebildete Rastabschnitte 255 und anders ausgebildete Rastaussparungen 259. Die weiteren Merkmale des Spritzenverschlusses 201 sind im Wesentlichen gleich den entsprechenden Merkmalen des Spritzenverschlusses 101, so dass insoweit auf obige Beschreibung verwiesen wird.

Die Rastabschnitte 255 der Verschlusskappe 217 sind in einem Bereich der Außenfläche 216 der Verschlusskappe 217 angeordnet, der vollständig von der Befestigungswand 223 umschlossen ist. Sie erstrecken sich ausgehend von der Außenfläche 216 der Verschlusskappe 217 in bezüglich der Hohlzylinderform der Verschlusskappe 217 radialer Richtung. Zusätzlich ist vorgesehen, daß eine Querschnittsfläche des jeweiligen Rastabschnitts 255, die senkrecht zur radialen Richtung orientiert ist, umso größer ist, je weiter sie von der Außenfläche der Verschlusskappe 217 entfernt ist (Hinterschneidungswirkung in radialer Richtung).

Die Rastabschnitte 255 sind formschlüssig von Rastaussparungen 259 umschlossen, die in der Befestigungswand 223 des Befestigungsrings 215 ausgebildet sind. Auch die Querschnittsfläche der Rastaussparung 259, die senkrecht zur radialen Richtung orientiert ist, vergrößert sich mit zunehmendem Abstand von der Außenfläche der Verschlusskappe 217. Die Rastaussparungen 259 durchdringen die Befestigungswand 223 des Befestigungsrings 215 vollständig, so dass die Rastabschnitte 255 von außen sichtbar sind. Die Rastabschnitte 255 enden bündig mit der Außenfläche der Befestigungswand 223.

Die Verschlusskappe 217 weist eine umlaufende Ringwulst 253 auf, die zwischen den Gewindeelementen 249 und den Längsrippen 243 der Verschlusskappe 217 angeordnet ist. Die Ringwulst 253 liegt an einer stimseitigen Endfläche 261 der Befestigungswand 223 an.

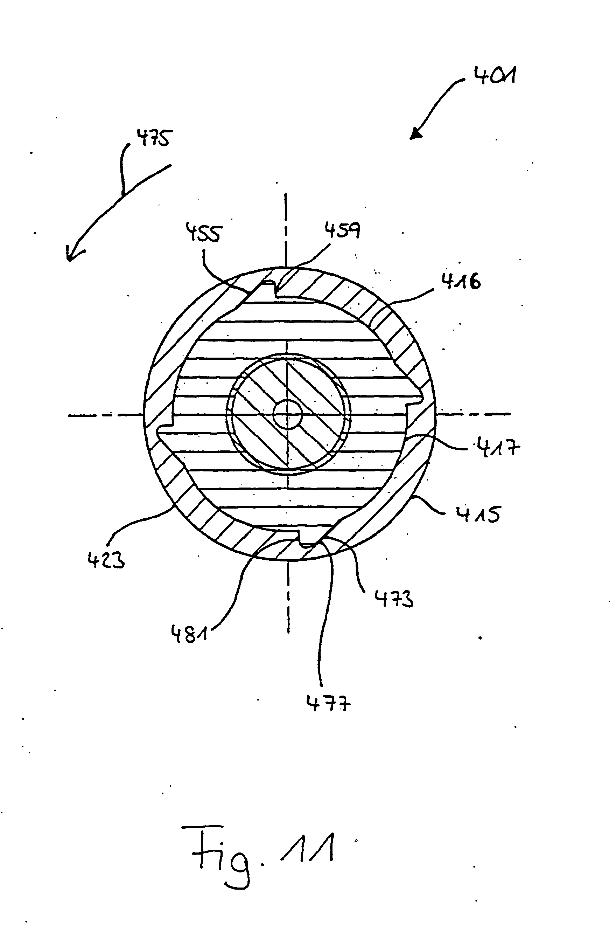

Figuren 6 bis 8 zeigen eine weitere Ausführungsform eines Spritzenverschlusses 301 mit einem Befestigungsring 315, einer Verschlusskappe 317 und einem Verschlusspfropfen 335. Dieser Spritzenverschluss 301 unterscheidet sich von den bisher erläuterten Spritzenverschlüssen 101 bzw. 201 durch anders ausgebildete Rastabschnitte 355 und Rastaussparungen 359. Die weiteren Merkmale des Spritzenverschlusses 301 sind im Wesentlichen gleich den entsprechenden Merkmalen der Spritzenverschlüsse 101 und 201, so dass insoweit auf obige Beschreibungen verwiesen wird.

Die Verschlusskappe 317 weist eine umlaufende Ringwulst 353 auf, die zwischen den Gewindeelementen 349 und den Längsrippen 343 der Verschlusskappe 317 angeordnet ist. Ausgehend von dieser Ringwulst 353 erstrecken sich vier Rastabschnitte 355 in proximaler Richtung, parallel zur Längsachse 357 und beabstandet zur Außenfläche 316 der Verschlusskappe 317, wobei die Rastabschnitte 355 mit zunehmenden Abstand von der Ringwulst 353 einen abnehmenden Querschnitt aufweisen und benachbarte Rastabschnitte 155 jeweils einen gleichen Winkelabstand zueinander aufweisen. Alternativ könnten auch mehr oder weniger als vier Rastabschnitte 355 ausgebildet sein.

Eine abgeschrägte Anschlagfläche 373 eines jeden Rastabschnitts 355 übt beim Herausdrehen in Drehrichtung 375 (in Fig. 6 von oben gesehen im Gegenuhrzeigersinn) einen Druck gegen eine gegenüberliegende, entsprechend abgeschrägte Seitenfläche 377 einer Rastaussparung 359 des Befestigungsrings 315 aus. Die Druckfläche weist einen Steigungswinkel α auf, der größer ist als eine Gewindesteigung, die durch die Gewindeelemente 349 festgelegt ist, so dass die Rastabschnitte 355 und/oder die Rastaussparungen 359 beim Herausdrehen der Verschlusskappe 317 aus dem Befestigungsring 315 zwangsläufig deformiert und Manipulationen an der Spritzenfüllung sichtbar werden. Auch diese Rastabschnitte 355 und Rastaussparungen 359 greifen derart ineinander bzw. sind gewissermaßen ineinander verrastet, so daß sie nur in einem Zweikomponenten-Spritzverfahren herstellbar sind.

Die Rastaussparungen 359 sind in der Befestigungswand 323 des Befestigungsrings 315 ausgebildet und umschließen die Rastabschnitte 355 formschlüssig. Die Rastaussparungen 359 sind zu einer stirnseitigen distalen Endfläche 361 hin geöffnet, und ihr Querschnitt verkleinert sich mit zunehmenden Abstand von der Endfläche 361. Die Rastaussparungen 359 gehen durch die Befestigungswand 323 hindurch, so dass die Rastabschnitte 355 für einen Benutzer von außen sichtbar sind. Von außen sichtbare Außenflächen der Rastabschnitte 355 sind bündig mit der Außenfläche der Befestigungswand 323.

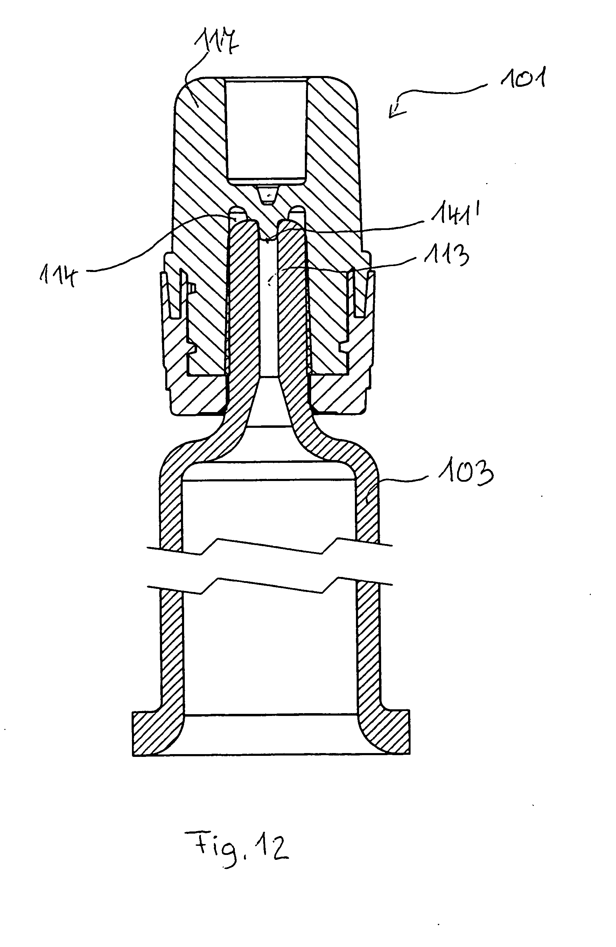

Figuren 9 bis 11 zeigen eine weitere Ausführungsform eines Spritzenverschlusses 401 mit einem Befestigungsring 415, einer Verschlusskappe 417 und einem Verschlusspfropfen 435. Dieser Spritzenverschluss 401 unterscheidet sich von den in den Figuren 1 bis 8 dargestellten Spritzenverschlüssen 101, 201 und 301 durch nochmals anders ausgebildete Rastabschnitte 455 und Rastaussparungen 459. Die sonstigen Merkmale des Spritzenverschlusses 401 entsprechen denen der Spritzenverschlüsse 101, 201 und 301, so dass insoweit auf obige Beschreibungen verwiesen wird.

Die im Querschnitt (Fig. 11) keilförmigen oder abgerundet dreieckförmigen Rastabschnitte 455 der Verschlusskappe 417 sind der Außenfläche 416 der Verschlusskappe 417 radial vorstehend angeordnet und von der Befestigungswand 423 des Befestigungsrings 415 vollständig umschlossen.

Eine Druckfläche 473 eines jeden Rastabschnitts 455 verläuft radial-tangential ansteigend, und eine Seitenfläche 481 eines jeden Rastabschnitts 455, die der Druckfläche 473 gegenüber liegt, verläuft in einer zu der Längsachse 157 parallelen oder diese enthaltenden Ebene. Beim Herausdrehen der Verschlusskappe 417 aus dem Befestigungsring 415 in Drehrichtung 475 (in Fig. 9 von oben gesehen im Gegenuhrzeigersinn) werden die Rastabschnitte 455 gegen entsprechende Seitenflächen 477 der Rastaussparungen 459 des Befestigungsrings 415 gedrückt und dabei, ggf. zusammen mit den Rastaussparungen 459, deformiert bzw. zerstört.

Die Rastaussparungen 459 umschließen die Rastabschnitte 455 formschlüssig und sind komplementär zu diesen in der Befestigungswand 423 des Befestigungsrings 415 ausgebildet. Die Rastaussparungen 459 sind lediglich Vertiefungen in der Innenfläche der Befestigungswand 423 und durchdringen diese nicht vollständig.

Die Verschlusskappe 417 weist eine umlaufende Ringwulst 453 auf, die zwischen den Gewindeelementen 449 und den Längsrippen 443 der Verschlusskappe 417 angeordnet ist. Die Ringwulst 453 liegt an einer stirnseitigen Endfläche 461 der Befestigungswand 423 an.

Der Ausdruck "verrastet" und ähnliche Ausdrücke beziehen sich im Rahmen der Erfindung auf räumlich derart ineinander greifende und einander hintergreifende, in Längsrichtung hinterschneidungsartig ausgebildete Ausformungen (Rastmittel), insbesondere ineinander greifende Rastabschnitte und Rastaussparungen, dass die Rastmittel nicht beschädigungsfrei voneinander lösbar sind. Umgekehrt kann der erfindungsgemäße Spritzverschluß nicht durch getrenntes Herstellen von Befestigungsring und Verschlußkappe und anschließendes Zusammenfügen und Verrasten hergestellt werden, da auch hierbei die Rastmittel erkennbar beschädigt oder zerstört würden. Eine Herstellung ist wie erläutert sinnvollerweise nur im Zweikomponenten-Spritzverfahren möglich.

Fig. 12 zeigt eine Variante der Abdichtung des Spritzenkörpers 103. Zur Abdichtung dient die Verschlusskappe 117, die hierzu einen der distalen Öffnung 113 des Spritzenkörpers 103 zugeordneten Abschnitt 141' zum Eingreifen in die distale Öffnung 113 aufweist. Auch ohne den Verschlusspfropfen 135 ist eine zuverlässige Abdichtung gewährleistet. Die Ausnehmung 114 kann als Ringnut oder auch mehrfach als Einzelnut zur Erhöhung der Elastizität angeordnet sein.

### Bezugszeichenliste:

- 101,201,301,401: Spritzenverschluss
- 103: Spritzenkörper
- 105: proximaler Endabschnitt des Spritzenkörpers
- 107: distaler Endabschnitt des Spritzenkörpers
- 109: Hohlzylinder
- 111: distale Spitze
- 113: distale Öffnung
- 114: Ausnehmung
- 115,215,315,415: Befestigungsring
- 116,216,316,416: Außenfläche (von 117)
- 117,217,317,417: Verschlusskappe
- 119: ringförmiger Abschnitt (von 115)
- 123, 223, 323, 423: hohlzylinderförmige Befestigungswand
- 125: Innenfläche (von 123, 223, 423)
- 127: Gewindeelemente (von 115)
- 129: proximaler Abschnitt (von 117)
- 131: distaler Abschnitt (von 117)
- 135, 235, 335, 435: Verschlusspfropfen
- 137: proximale Endfläche (von 135)
- 141: vorstehender Abschnitt (von 135)
- 141': vorstehender Abschnitt an 117
- 143, 243, 343, 443: Längsrippen
- 149, 349, 449: Gewindeelemente (von 117)
- 153,253,353,453: Ringwulst
- 155, 255, 355, 455: Rastabschnitte
- 157: Längsachse
- 159,259,359,459: Rastaussparungen
- 161, 261, 361, 461: distale, stirnseitige Endfläche (von 115)
- 373,473: Anschlagfläche
- 375,475: Drehrichtung
- 377, 477: Seitenfläche (von 359)
- 381, 481: Seitenfläche (von 355)

- α: Steigungswinkel (von 373, 473)

## Patentansprüche

1. Spritzenverschluss zum abdichtenden Verschließen einer distalen Öffnung (113) eines Spritzenkörpers (103), wobei der Spritzenverschluss (101; 201; 301; 401) einen an dem Spritzenkörper (103) um die distale Öffnung (111) herum anordbaren und befestigbaren Befestigungsring (115; 215; 315; 415) und eine die distale Öffnung (113) abdichtend verschließende und mit dem Befestigungsring (115; 215; 315; 415) lösbar verbundene Verschlusskappe (117; 217; 317; 417) aufweist, **dadurch gekennzeichnet, dass** der Befestigungsring (115; 215; 315; 415) ein Rastmittel (155; 255; 355; 455) umfasst, das mit einem Rastmittel (159; 259; 359; 459) der Verschlusskappe (117; 217; 317; 417) verrastet ist, und wobei das Rastmittel (155; 255; 355; 455) des Befestigungsrings (115; 215; 315; 415) und das Rastmittel (159; 259; 359; 459) der Verschlusskappe (117; 217; 317; 417) so ausgebildet sind, dass sie nicht beschädigungsfrei entrastbar sind und nach Entrasten nicht wieder in ihren Ausgangszustand zu bringen sind.

2. Spritzenverschluss nach Anspruch 1, **dadurch gekennzeichnet, dass** die Rastmittel durch Rastabschnitte (155; 255; 355; 455) und diese formschlüssig umschließende Rastaussparungen (159; 259; 359; 459) gebildet sind, wobei die Rastabschnitte (155; 255; 355; 455) entweder mit dem Befestigungsring (115; 215; 315; 415) oder mit der Verschlusskappe (117; 217; 317; 417) und die Rastaussparungen (159; 259; 359; 459) mit dem anderen Teil, Befestigungsring (115; 215; 315; 415) oder Verschlusskappe (117; 217; 317; 417), verbunden sind, so dass die Verschlusskappe (117; 217; 317; 417) mit dem Befestigungsring (115; 215; 315; 415) verrastet ist, nicht beschädigungsfrei entrastbar ist und nach Entrasten nicht wieder in ihren Ausgangszustand zu bringen sind.

3. Spritzenverschluss nach Anspruch 2, **dadurch gekennzeichnet, dass** die Rastaussparungen (155) in Längsrichtung hinterschnitten ausgebildet sind.

4. Spritzenverschluss nach einem der vorhergehenden Ansprüche, **dadurch gekennzeichnet, dass** die Rastaussparungen (255) in radialer Richtung hinterschnitten ausgebildet sind.

5. Spritzenverschluss nach einem der vorhergehenden Ansprüche, **dadurch gekennzeichnet, dass** der Befestigungsring (115) einen proximalen, ringförmigen Abschnitt (119) und eine sich distal von dem ringförmigen Abschnitt (119) erstreckende, hohlzylinderförmige Befestigungswand (123) aufweist.

6. Spritzenverschluss nach Anspruch 5, soweit rückbezogen auf Anspruch 2, **dadurch gekennzeichnet, dass** die Rastabschnitte (155) mit einer äußeren Ringwulst (153) der Verschlusskappe (117) verbunden sind und sich ausgehend von der Ringwulst (153) parallel zur Längsachse (157), in proximaler Richtung und mit Abstand zu einer Außenfläche der Verschlusskappe (117) erstrecken, wobei die Rastaussparungen (159) zu einer distalen, stirnseitigen Endfläche (161) der Befestigungswand (123) hin geöffnet sind.

7. Spritzenverschluss nach Anspruch 5, **dadurch gekennzeichnet, dass** in der Befestigungswand (223; 423) Rastaussparungen (259; 459) ausgebildet sind, die auf einer Außenfläche (216; 416) der Verschlusskappe (217; 417) ausgebildete Rastabschnitte (255; 455) formschlüssig umschließen.

8. Spritzenverschluss nach einem der vorhergehenden Ansprüche, **dadurch gekennzeichnet, dass** der Befestigungsring (115) und die Verschlusskappe (117) miteinander zusammenwirkende Gewindeelemente (127, 149) aufweisen, die eine Gewindesteigung festlegen.

9. Spritzenverschluss nach Anspruch 8, soweit rückbezogen auf Anspruch 2, **dadurch gekennzeichnet, dass** jeder Rastabschnitt (355) eine unter einem Steigungswinkel (α) verlaufende Anschlagfläche (373) aufweist, die bei einem durch die Gewindeelemente (349) geführten Herausdrehen der Verschlusskappe (317) aus dem Befestigungsring (315) gegen eine Seitenfläche (381) der den jeweiligen Rastabschnitt (355) umschließenden Rastaussparung (459) anliegt, wobei der Steigungswinkel (α) größer ist als die Gewindesteigung, so dass die Rastabschnitte (315) und/oder die Rastaussparungen (317) beim Herausdrehen zwangsläufig deformiert werden.

10. Spritzenverschluss nach einem der vorhergehenden Ansprüche, **dadurch gekennzeichnet, dass** der Befestigungsring (115; 215; 315; 415) auf eine distale Spitze (111) des Spritzenkörpers (103), durch die sich die distale Öffnung (113) erstreckt, aufsetzbar und darauf fixierbar ist.

11. Spritzenverschluss nach einem der vorhergehenden Ansprüche, **dadurch gekennzeichnet, dass** die Verschlusskappe (117) zum Aufnehmen einer distalen Spitze (111) des Spritzenkörpers (103) die Form eines einseitig geschlossenen Hohlzylinders aufweist.

12. Spritzenverschluss nach Anspruch 11, **dadurch gekennzeichnet, dass** in der Verschlusskappe (117) ein zylinderförmiger Verschlusspfropfen (135) angeordnet ist, auf der ein mittiger, vorstehender Abschnitt (141) zum Eingreifen in die distale Öffnung (113) des Spritzenkörpers (103) angeordnet ist.

13. Spritzenverschluss nach Anspruch 11, **dadurch gekennzeichnet, dass** die Verschlusskappe (117) einen der distalen Öffnung (113) des Spritzenkörpers (103) zugeordneten mittigen Abschnitt (141') zum Eingreifen in die distale Öffnung (113) aufweist.

14. Spritzenverschluss nach einem der vorhergehenden Ansprüche, **dadurch gekennzeichnet, dass** die Verschlusskappe (117) äußere Längsrippen (143) aufweist.

15. Spritzenverschluss nach einem der vorhergehenden Ansprüche, **dadurch gekennzeichnet, dass** die Verschlusskappe (117) aus thermoplastischen Elastomeren gebildet ist.

16. Spritzenverschluss nach einem der vorhergehenden Ansprüche, **dadurch gekennzeichnet, dass** der Befestigungsring (115; 215; 315; 415) und die Verschlusskappe (117; 217; 317; 417) in einem Zweikomponenten-Spritzverfahren aus unterschiedlichen, sich nicht einstückig miteinander verbindenden Materialien hergestellt sind.

17. Verfahren zum Herstellen eines Spritzenverschlusses nach einem der vorhergehenden Ansprüche, bei dem die Verschlusskappe (117; 217; 317; 417) und der Befestigungsring (115; 215; 315; 415) zusammen mittels eines Zweikomponenten-Spritzverfahrens so ineinander verrastet hergestellt werden, dass sie nicht beschädigungsfrei entrastbar sind und nach Entrasten nicht wieder beschädigungsfrei gefügt werden können.

18. Verfahren nach Anspruch 17, **dadurch gekennzeichnet, dass** nach Herstellung der Verschlusskappe (117; 217; 317; 417) und des Befestigungsrings (115; 215; 315; 415) ein Verschlusspfropfen (135; 235; 335; 435) in die Verschlusskappe (117; 217; 317; 417) eingesetzt wird.

## Claims

1. Syringe closure for the sealing closing of a distal opening (113) of a syringe body (103), wherein the syringe closure (101; 201; 301; 401) comprises a fastening ring (115; 215; 315; 415), which can be arranged at and fastened to the syringe body (103) around the distal opening (111), and a closure cap (117; 217; 317; 417), which sealingly closes the distal opening (113) and is detachably connected with the fastening ring (115; 215; 315; 415), **characterised in that** the fastening ring (115; 215; 315; 415) comprises a detent means (155; 255; 355; 455) detented with a detent means (159; 259; 359; 459) of the closure cap (117; 217; 317; 417) and wherein the detent means (155; 255; 355; 455) of the fastening ring (115; 215; 315; 415) and the detent means (159; 259; 359; 459) of the closure cap (117; 217; 317; 417) are so constructed that they cannot be released from detenting without damage and after release from detenting cannot be returned to the initial state thereof.

2. Syringe closure according to claim 1, **characterised in that** the detent means are formed by detent sections (155; 255; 355; 455) and detent cut-outs (159; 259; 359; 459) surrounding these in shape-locking manner, wherein the detent sections (155; 255; 355; 455) are connected either with the fastening ring (115; 215; 315; 415) or with the closure cap (117; 217; 317; 417) and the detent cut-outs (159; 259; 359; 459) with the other part, i.e. fastening ring (115; 215; 315; 415) or closure cap (117; 217; 317; 417), so that the closure cap (117; 217; 317; 417) is detented with the fastening ring (115; 215; 315; 415), cannot be released from detenting without damage and after release from detenting cannot be returned to the initial state thereof.

3. Syringe closure according to claim 2, **characterised in that** the detent cut-outs (155) are formed to be undercut in longitudinal direction.

4. Syringe closure according to one of the preceding claims, **characterised in that** the detent cut-outs (255) are formed to be undercut in radial direction.

5. Syringe closure according to one of the preceding claims, **characterised in that** the fastening ring (115) has a proximal annular section (119) and a distal hollow-cylindrical fastening wall (123) extending from the annular section (119).

6. Syringe closure according to claim 5 when appended to claim 2, **characterised in that** the detent sections (155) are connected with an outer annular bead (153) of the closure cap (117) and, starting from the annular bead (153), extend parallel to the longitudinal axis (157) in proximal direction and at a spacing from an outer surface of the closure cap (117), wherein the detent cut-outs (159) are open towards a distal end surface (161), which is at the end face, of the fastening wall (123).

7. Syringe closure according to claim 5, **characterised in that** detent cut-outs (259; 459), which in shape-locking manner surround detent sections (255; 455) formed on an outer surface (216; 416) of the closure cap (217; 417), are formed in the fastening wall (223; 423).

8. Syringe closure according to one of the preceding claims, **characterised in that** the fastening ring (115) and the closure cap (117) comprise mutually co-operating threaded elements (127, 149) determining a thread pitch.

9. Syringe closure according to claim 8 when appended to claim 2, **characterised in that** each detent section (355) has an abutment surface (373), which extends at an angle (α) of inclination and which bears against a side surface (381) of the detent cut-out (459) surrounding the respective detent section (355) when the closure cap (317) is turned out of the fastening ring (315) under the guidance of the threaded element (349), wherein the angle (α) of inclination is greater than the thread pitch so that the detent sections (315) and/or the detent cut-outs (317) are forcibly deformed during the turning out.

10. Syringe closure according to one of the preceding claims, **characterised in that** the fastening ring (115; 215; 315; 415) can be placed on a distal tip (111) of the syringe body (103), through which the distal opening (113) extends, and fixed thereon.

11. Syringe closure according to one of the preceding claims, **characterised in that** the closure cap (117) has the shape of a hollow cylinder, which is closed at one end, for reception of a distal tip (111) of the syringe body (103).

12. Syringe closure according to claim 11, **characterised in that** a cylindrical closure plug (135), on which a central, protruding section (141) for engagement in the distal opening (113) of the syringe body (103) is arranged, is arranged in the closure cap (117).

13. Syringe closure according to claim 11, **characterised in that** the closure cap (117) has a central section (141'), which is associated with the distal opening (113) of the syringe body (103), for engagement in the distal opening (113).

14. Syringe closure according to one of the preceding claims, **characterised in that** the closure cap (117) has external longitudinal ribs (143).

15. Syringe closure according to one of the preceding claims, **characterised in that** the closure cap (117) is formed from thermoplastic elastomer.

16. Syringe closure according to one of the preceding claims, **characterised in that** the fastening ring (115; 215; 315; 415) and the closure cap (117; 217; 317; 417) are produced in a two-component injection-moulding method from different materials which do not integrally connect together.

17. Method of producing a syringe closure according to one of the preceding claims, in which the closure cap (117; 217; 317; 417) and the fastening ring (115; 215; 315; 415) are produced by means of a two-component injection-moulding method to be so detented in one another that they cannot be released from detenting without damage and after release of detenting cannot be joined together again without damage.

18. Method according to claim 17, **characterised in that** a closure plug (135; 235; 335; 435) is inserted in the closure cap (117; 217; 317; 417) after production of the closure cap (117; 217; 317; 417) and the fastening ring (115; 215; 315; 415).

## Revendications

1. °) Capuchon de seringue destiné à fermer de manière étanche une ouverture distale (113) d'un corps de seringue (103), le capuchon de seringue (101 ; 201 ; 301, 401) présentant une bague de fixation (115 ; 215 ; 315 ; 415) qui peut être placée et fixée sur le corps de seringue (103) autour de l'ouverture distale (113) et un capuchon de fermeture (117 ; 217 ; 317 ; 417) fermant l'ouverture distale (113) de manière étanche et relié de manière à pouvoir être séparé à la bague de fixation (115 ; 215 ; 315 ; 415),
**caractérisé en ce que**
la bague de fixation (115; 215 ; 315 ; 415) comprend un moyen d'accrochage (155 ; 255 ; 355 ; 455) qui s'accroche avec un moyen d'accrochage (159 ; 259 ; 359 ; 459) du capuchon de fermeture (117 ; 217 ; 317 ; 417), et le moyen d'accrochage (155 ; 255 ; 355 ; 455) de la bague de fixation (115 ; 215 ; 315 ; 415) et le moyen d'accrochage (159 ; 259 ; 359 ; 459) du capuchon de fermeture (117 ; 217 ; 317 ; 417) sont formés de manière à ne pas pouvoir se décrocher sans risque d'endommagement, et après le décrochage, à ne pas pouvoir être remis dans leur état de départ.

2. °) Capuchon de seringue selon la revendication 1,
**caractérisé en ce que**
les moyens d'accrochage sont formés par des parties d'accrochage (155 ; 255 ; 355 ; 455) et des évidements d'accrochage (159 ; 259 ; 359 ; 459) qui les entourent par complémentarité de forme, les parties d'accrochage (155 ; 255 ; 355 ; 455) sont reliées soit à la bague de fixation (115 ; 215 ; 315 ; 415) soit au capuchon de fermeture (117 ; 217 ; 317 ; 417) et les évidements d'accrochage (159 ; 259 ; 359 ; 459) avec l'autre partie, bague de fixation ou capuchon de fermeture, de façon à ce que le capuchon de fermeture (117 ; 217 ; 317 ; 417) soit accroché avec la bague de fixation et qu'après le désaccrochage, ils ne puissent pas être remis dans leur état de départ.

3. °) Capuchon de seringue selon la revendication 2,
**caractérisé en ce que**
les évidements d'accrochage (159) sont formés à la manière d'une contre-dépouille longitudinale.

4. °) Capuchon de seringue selon l'une des revendications précédentes,
**caractérisé en ce que**
les évidements d'accrochage (259) sont formés à la manière d'une contre-dépouille dans le sens radial.

5. °) Capuchon de seringue selon l'une des revendications précédentes,
**caractérisé en ce que**
la bague de fixation (115) présente une partie proximale annulaire (119) et une paroi de fixation cylindrique creuse (123) s'étendant de manière distale depuis la partie annulaire (119).

6. °) Capuchon de seringue selon la revendication 5, si l'on se réfère à la revendication 2,
**caractérisé en ce que**
les parties d'accrochage (155) sont reliées à un bourrelet annulaire extérieur (153) du capuchon de fermeture (117) et s'étendent depuis le bourrelet annulaire (153) parallèlement à l'axe longitudinal (157), dans le sens proximal et à distance d'une surface extérieure du capuchon de fermeture (117), moyennant quoi les évidements d'accrochage (159) sont ouverts sur une surface d'extrémité distale frontale (161) de la paroi de fixation (123).

7. °) Capuchon de seringue selon la revendication 5,
**caractérisé en ce que**
dans la paroi de fixation (223 ; 423) on forme des évidements d'accrochage (259 ; 459) qui entourent par complémentarité de forme des parties d'accrochage (255 ; 455) formées sur une surface extérieure (216 ; 416) du capuchon de fermeture (217, 417).

8. °) Capuchon de seringue selon l'une des revendications précédentes,
**caractérisé en ce que**
la bague de fixation (115) et le capuchon de fermeture (117) présentent des éléments de filetage (127, 149) coopérant entre eux qui définissent un pas de vis.

9. °) Capuchon de seringue selon la revendication 8, si l'on se réfère à la revendication 2,
**caractérisé en ce que**
chaque partie d'accrochage (355) présente une surface de butée (373) se profilant dans un angle du pas de vis (α) qui, lorsque le capuchon de fermeture (317) est dévissé de la bague de fixation (315) par les éléments de filetage (349), s'appuie contre une surface latérale (381) de l'évidement d'accrochage (459) entourant la partie d'accrochage (355) respective, dans lequel l'angle du pas de vis (α) est supérieur au pas de vis, de façon à ce que les parties d'accrochage (315) et/ou les évidements d'accrochage (317) soient obligatoirement déformés lors du dévissage.

10. °) Capuchon de seringue selon l'une des revendications précédentes,
**caractérisé en ce que**
la bague de fixation (115; 215 ; 315 ; 415) peut être placée sur une pointe distale (111) du corps de seringue (103) à travers laquelle l'ouverture distale (113) s'étend, et peut être fixée dessus.

11. °) Capuchon de seringue selon l'une des revendications précédentes,
**caractérisé en ce que**
le capuchon de fermeture (117) se présente sous la forme d'un cylindre creux fermé sur un côté pour recevoir une pointe distale (111) du corps de seringue (103).

12. °) Capuchon de seringue selon la revendication 11,
**caractérisé en ce que**
dans le capuchon de fermeture (117) est placé un bouchon de fermeture cylindrique (135), sur lequel une partie en saillie centrale (141) est placée pour s'engager dans l'ouverture distale (113) du corps de seringue (103).

13. °) Capuchon de seringue selon la revendication 11,
**caractérisé en ce que**
le capuchon de fermeture (117) présente une partie (141') par rapport à l'ouverture distale (113) du corps de seringue (103) pour s'engager dans l'ouverture distale (113).

14. °) Capuchon de seringue selon l'une des revendications précédentes,
**caractérisé en ce que**
le capuchon de fermeture (117) présente des nervures longitudinales extérieures (143).

15. °) Capuchon de seringue selon l'une des revendications précédentes,
**caractérisé en ce que**
le capuchon de fermeture (117) est en élastomère thermoplastique.

16. °) Capuchon de seringue selon l'une des revendications précédentes,
**caractérisé en ce que**
la bague de fixation ( 115 ; 215 ; 315 ; 415) et le capuchon de fermeture (117 ; 217 ; 317 ; 417) sont fabriqués selon un procédé par injection de deux composants dans des matériaux différents qui ne se lient pas entre eux d'une seule pièce.

17. °) Procédé de fabrication d'un capuchon de seringue selon l'une des revendications précédentes, selon lequel le capuchon de fermeture (117 ; 217 ; 317 ; 417) et la bague de fixation (115 ; 215 ; 315 ; 415) sont fabriqués ensemble par accrochage l'un dans l'autre au moyen d'un procédé par injection de deux composants, de façon à ne pas pouvoir être désaccrochés sans risque d'endommagement et à ne pas pouvoir être de nouveau assemblés sans risque d'endommagement après le désaccrochage.

18. °) Procédé selon la revendication 17,
**caractérisé en ce qu'**
après la fabrication du capuchon de fermeture (117 ; 217 ; 317 ; 417) et de la bague de fixation (115 ; 215 ; 315 ; 415), un bouchon de fermeture (135 ; 235 ; 335 ; 345) est inséré dans le capuchon de fermeture (117 ; 217 ; 317;417).
